# EUROPEAN PATENT APPLICATION

(11) **EP 4 449 987 A1**
(43) Date of publication of application: **23.10.2024**
(21) Application number: 22906976.0
(22) Date of filing: 06.10.2022
(51) Int. Cl.: A61B 5/107, G06T 11/80

(54) **IMAGE DISPLAY DEVICE, IMAGE DISPLAY METHOD, PROGRAM, AND STORAGE MEDIUM**

(30) Priority: 17.12.2021 JP 2021205315
(71) Applicant: CANON KABUSHIKI KAISHA, Tokyo 146-8501 (JP)
(72) Inventor: IKEMOTO, Kiyokatsu, Tokyo 146-8501 (JP); MORI, Shingo, Tokyo 146-8501 (JP)
(74) Representative: TBK
(86) International application number: PCT/JP2022/037447
(87) International publication number: WO 2023/112441

(57) **Abstract**

An image display device for improvement in display of an image is provided. An image display device includes: acquisition means for acquiring a first image, a second image, and first position information regarding a first position at which a subject in the first image is measured; display means for displaying the second image on a display device; and determination means for determining a second position corresponding to the first position in the second image. The display means displays and superimposes first measurement information obtained by measuring a subject at the first position on the first image at the second position on the second image.

## Description

### [Technical Field]

The present invention relates to an image display device, an image display method, a program, and a storage medium.

### [Background Art]

Techniques for measuring dimensions of subjects in images by acquiring information regarding distances between imaging devices and the subjects when the images are captured have been proposed. PTL 1 proposes a method of detecting dimension measurement positions designated by a user on an image and measuring dimensions between the designated positions. PTL 1 also discloses that information regarding dimensions measured in images is superimposed on an image to be displayed side by side.

Such an image display method can be used for confirming a skin disease part over time by displaying an image obtained by imaging the skin disease part periodically, for example, in a medical site along with information regarding dimensions of the disease part. The image display method can also be used for observing infrastructure inspection such as crack or deformation of a structure over time. Alternatively, the image display method can also be used for confirming a difference between sizes of substantially the same subjects (for example, individual differences between animals or plants or individual differences between used products or artifacts in electronic commerce).

### [Citation List]

### [Patent Literature]

[PTL 1]
Japanese Patent Laid-open No. 2019-158519

### [Summary of Invention]

### [Technical Problem]

In the scheme of the related art, however, a measurement result in each image is displayed on each image, it is necessary to compare each image and confirm a difference in size, and therefore it is difficult to ascertain the difference. When substantially the same positions on a subject are measured, it is difficult to designate substantially the same positions on each image if a direction or a size of the subject of each image is different.

As described above, there is room for improvement in display of an image.

The present invention has been made in view of the foregoing problems and an object of the present invention is to provide an image display device, an image display method, a program, and a storage medium for improvement in display of an image.

### [Solution to Problem]

According to an aspect of the present invention, an image display device includes: acquisition means for acquiring a first image, a second image, and first position information regarding a first position at which a subject in the first image is measured; display means for displaying the second image on a display device; and determination means for determining a second position corresponding to the first position in the second image. The display means displays and superimposes first measurement information obtained by measuring a subject at the first position on the first image at the second position of the second image.

### [Advantageous Effects of Invention]

According to the present invention, it is possible to provide an image display device, an image display method, a program, and a storage medium for improvement in display of an image.

### [Brief Description of Drawings]

Fig. 1 is a block diagram illustrating a functional configuration for performing an image display method according to an embodiment of the present invention.
Fig. 2 is a flowchart illustrating an image display process performed in a computer 100 according to the embodiment.
Fig. 3 is a diagram illustrating an example of a screen displayed on an output unit 14 after a first image is selected.
Fig. 4 is a diagram illustrating an example of a method of designating a measurement position when an area is desired to be measured.
Fig. 5 is a diagram illustrating display in which information regarding a measurement position is superimposed in an expanded image 302 of a first image 301.
Fig. 6(A) is a diagram illustrating an example of a screen displayed on the output unit 14 after a second image 601 is selected and Fig. 6(B) is a flowchart illustrating a process of calculating a corresponding position of a subject in the first image 301 and the second image 601.
Fig. 7 is a diagram illustrating display in which sizes of images of the subject of the first image 301 and the second image 601 are substantially the same based on an imaging magnification.
Fig. 8(A) is a diagram illustrating an example of a screen displayed on the output unit 14 when positions and postures of the first image 301 and the second image 701 in imaging are slightly different and Fig. 8(B) is a flowchart illustrating a deformation process of expanding and contracting the second image in consideration of a difference in an inclination of a posture based on distance information.
Fig. 9 is a diagram illustrating an image 7012 that is an image obtained by deforming the second image 701 using a magnification calculated in S703.
Fig. 10(A) is a diagram illustrating an example of a screen displayed on the output unit 14 when positions and postures of the first image 301 and the second image 801 in imaging are significantly different and Fig. 10(B) is a flowchart illustrating a process of calculating corresponding positions based on distance information.
Fig. 11 is a diagram illustrating display in which information regarding a measurement position of the first image is superimposed on a corresponding position 403 of the second image and displayed on the output unit 14.
Fig. 12(A) is a diagram illustrating an expanded second image 602 and Fig. 12(B) is a diagram illustrating an image 6021 in which a new measurement position 4031 and information regarding a measurement position 4031 is superimposed.
Fig. 13 is a diagram illustrating an example when only a measurable range can be designated as a measurement position.
Fig. 14 is a diagram illustrating an example when any target is measured based on distance information.

### [Description of Embodiments]

Hereinafter, modes for carrying out the present invention will be described in detail with reference to the appended drawings. The following embodiments do not limit the present invention related to the claims. While a plurality of features are described in the embodiments, not all of the plurality of features are necessarily essential to the present invention, and furthermore, the plurality of features may be combined in any manner. Further, in the appended drawings, the same reference numerals are given to the same or similar configurations and repeated description will be omitted.

In the following embodiment, an image display device that is a device performing an example of an image display method according to the present invention with a computer will be described. In the image display method according to the present invention, for example, captured images acquired from an imaging device such as a digital camera are used. In the image display method according to the present invention, a plurality of pieces of distance information corresponding to the captured images are acquired, and dimension information of subjects captured in the captured images is combined with the captured images appropriately deformed for easy comparison to be displayed. A program for realizing the image display method according to the present invention can be executed in a desktop computer, a laptop computer, and a portable computer. The distance information corresponding to a certain image is an example of shape information indicating a shape of a subject captured in the certain image.

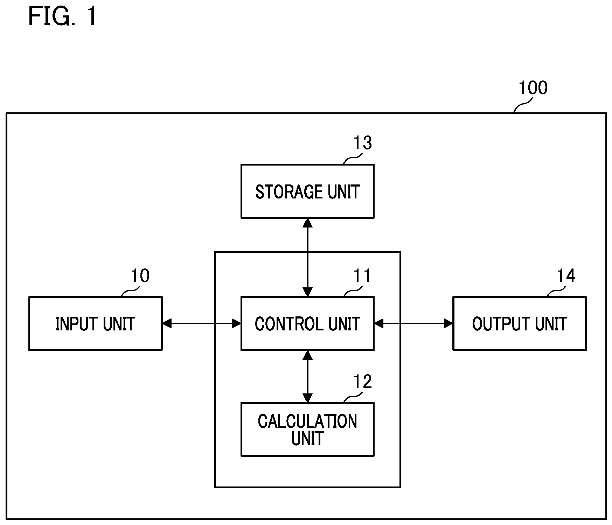

A configuration of a computer that is an example of an image display device performing the image display method according to an embodiment of the present invention will be described with reference to Fig. 1. Fig. 1 is a block diagram illustrating a functional configuration for performing an image display method according to the present invention. The image display device performing the image display method according to the present invention may include other constituent elements.

A computer 100 includes an input unit 10, a control unit 11, a calculation unit 12, a storage unit 13, and an output unit 14. The input unit 10 includes a user interface that acquires an action from a user, such as a mouse, a keyboard, or a joystick and an interface (I/F) that acquires an image and distance information from a digital camera or another external device.

The control unit 11 performs controls of the entire computer 100, such as control of the input unit 10, the calculation unit 12, the storage unit 13, and the output unit 14. The calculation unit 12 includes a calculation processor and executes a calculation processing program stored in the storage unit 13. The control unit 11 contains a CPU as a computer and performs control of each unit of the entire computer 100 via a bus based on a computer program stored in a nonvolatile memory. The CPU is an abbreviation for a central processing unit.

The storage unit 13 is configured with a primary storage such as a memory or a secondary storage such as a hard disk. The storage unit 13 stores various types of information such as image data, distance information, subject dimension information, and a parameter input via the input unit 10. The storage unit 13 stores an image generated through combination by the calculation unit 12.

The output unit 14 is, for example, a display and is an I/F that outputs a combined image.

The image display method according to the present invention may be performed by a PC or a portable computer realized by a touch panel display that serves for user interface functions of the input unit 10 and the output unit 14 of Fig. 1. At this time, the input unit 10 and the output unit 14 further includes an interface that acquires an image and distance information from a digital camera or another external device (not illustrated).

### (Image Display Method)

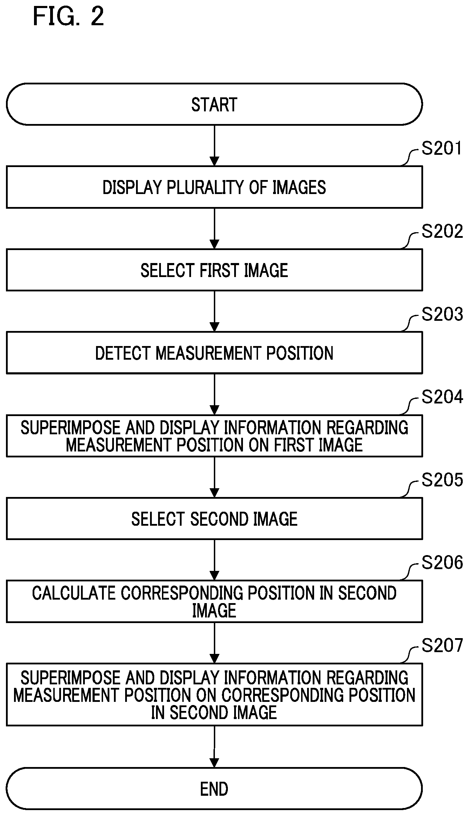

A specific process in an image display process performed in the computer 100 according to the embodiment will be described with reference to the flowchart of Fig. 2. A process corresponding to the flowchart is realized by causing the control unit 11 to read, for example, a corresponding processing program stored in a nonvolatile memory in the storage unit 13 and loading and executing the processing program in a volatile memory in the control unit 11.

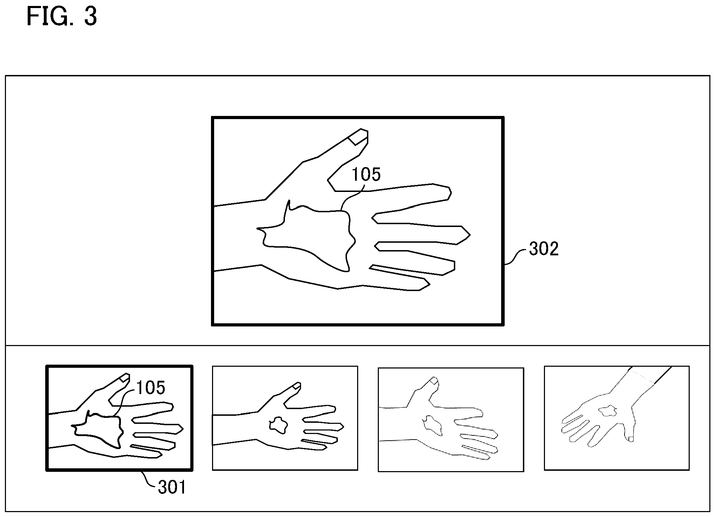

In S201, the control unit 11 displays a plurality of images stored in the storage unit 13 on the output unit 14. The images stored in the storage unit 13 are images to which distance information is added. The control unit 11 stores an image acquired via an imaging unit and a ranging unit (not illustrated) in the storage unit 13. The control unit 11 stores images captured by an external imaging device and images acquired via an interface of the input unit 10 in the storage unit 13. In the embodiment, images (see Fig. 3) of a skin disease part 105 occurring on a hand of a patient are used for description.

In S202, the control unit 11 selects a desired image (hereinafter referred to as a "first image") of a user via the input unit 10 from the plurality of displayed images.

Fig. 3 is a diagram illustrating an example of a screen displayed on the output unit 14 after the first image is selected. In Fig. 3, images listed in the lower portion are the plurality of images displayed in S201 and are images obtained by imaging the skin disease part 105 while changing camera parameters, an imaging position, and an imaging time. The user selects a first image 301 from the plurality of displayed images. The selected first image is displayed as an expanded image 302 with a large size in an intermediate portion to an upper portion of a screen. At this time, since a currently selected image is indicated so that it is easy to understand the image, an outer frame of the first image 301 may be displayed thicker than the other images or color of the frame may be changed.

In S203, the control unit 11 detects a measurement position desired by the user using the input unit 10 in the first image. Here, when the user desires to measure a length of a line between two certain points of a subject, the measurement position is positional information on an image on the two points or a line connecting the two points. When the user desires to measure an area of a certain region, the measurement position is positional information on an image of the region. The user designates a desired measurement position while viewing the displayed first image using an input device such as a mouse or a touch panel.

A method of designating a measurement position may be appropriately changed for each target which the user desires to measure. For example, when the user desires to measure a length between two points of a subject, the user may designate two points on an image. When the user desires to measure a length of a curve, the user may designate connected line segments connecting a plurality of points from a starting point to an ending point. When the user desires to measure an area, the user may designate a region surrounded by connecting the starting point to the ending point of the above-described curve.

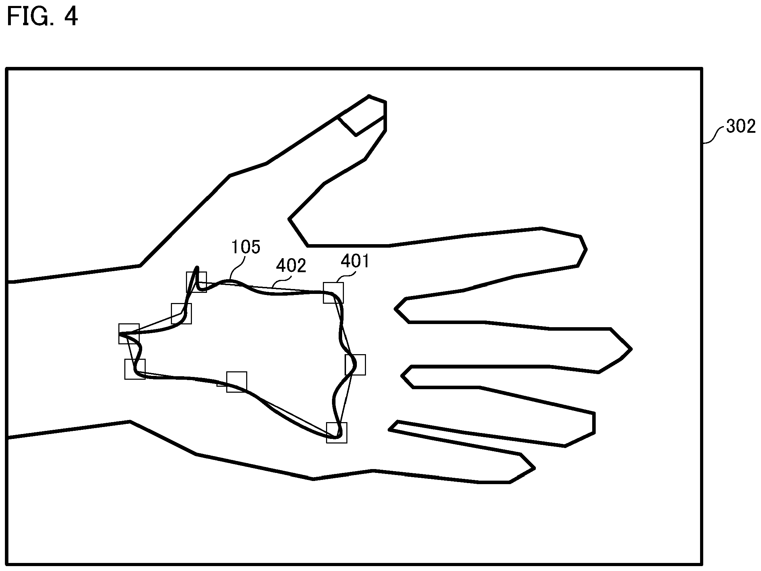

Fig. 4 is a diagram illustrating an example of a method of designating a measurement position when an area is desired to be measured. A measurement region 402 indicates a measurement position. Squares 401 indicate nodes for adjusting an outer circumferential shape of the measurement region 402. The user adjusts a shape of the figure 402 surrounded by a thin line by adjusting the positions of the nodes 401 on an expanded image 302 that is displayed to be large, and designates positional information on an image of a region which the user desires to measure. The expanded image 302 is divided into super-pixels and the user may designate a measurement position (measurement region) by designating the super-pixels.

A technique such as an AI or a neural network may be used to detect a specific subject region such as a disease part and set the positional information as a measurement position. By performing region detection by an AI technique using measurement positional information input by the user, a subject region may be detected with higher accuracy and the obtained positional information may be set as a measurement position. AI is an abbreviation for artificial intelligence.

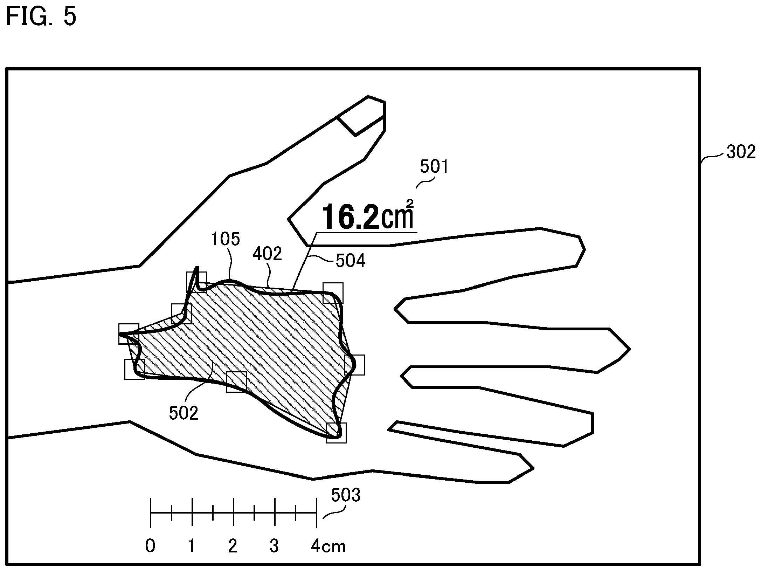

In S204, as illustrated in Fig. 5, the control unit 11 superimposes and displays information regarding the measurement position on the expanded image 302 of the first image 301 on the output unit 14. The information regarding the measurement position is information for showing a measurement result 501 such as an area or a length or a measurement portion such as an oblique line region 502 and further a scale 503 or an auxiliary line 504 suggesting the information to the user for easy understanding. The area or the length is calculated by an existing method based on distance information corresponding to the first image. The information regarding the measurement position is called measurement information and includes first measurement information and second measurement information. The first measurement information is information regarding a length of a line connecting two points including a first position on the first image or information regarding an area of a region including the first position on the first image. Alternatively, the first measurement information is obtained by measuring a length or an area of a subject based on the first image and first distance information in a depth direction corresponding to the first image. Alternatively, the first measurement information may include a line connecting two points including the first position or a frame indicating a region including the first position. The first measurement information preferably includes information regarding measurement accuracy when a subject is measured.

Here, the distance information is not limited to a distance between an imaging device and a subject and is information necessary to measure a subject, such as information indicating to which mm size one pixel corresponds in each pixel of an image or focal distance information in imaging. The distance information may also be a distance to an object recognized through object recognition or a distance value corresponding to a map of a layer obtained by decomposing an image into layers for each distance. The distance information may be first positional information or second positional information. The first positional information is information regarding the first position at which a subject in the first image is measured. The second positional information is information regarding a second position corresponding to the first position in the second image, as will be described below.

In S205, the control unit 11 selects an image desired by the user and different from the first image (hereinafter referred to as a "second image") through the input unit 10 in the plurality of displayed images. The number of selected second images may be only one or plural.

In S206, the control unit 11 calculates a position on an image corresponding to the measurement position of the first image (hereinafter referred to as a "corresponding position") in the second image. The control unit 11 performs the calculation based on the first image, the second image, and the distance information of these images. Hereinafter, a method of calculating the corresponding position will be described, but this method is merely exemplary. Another existing method may be used or any combination of suggested methods may be combined for the calculation.

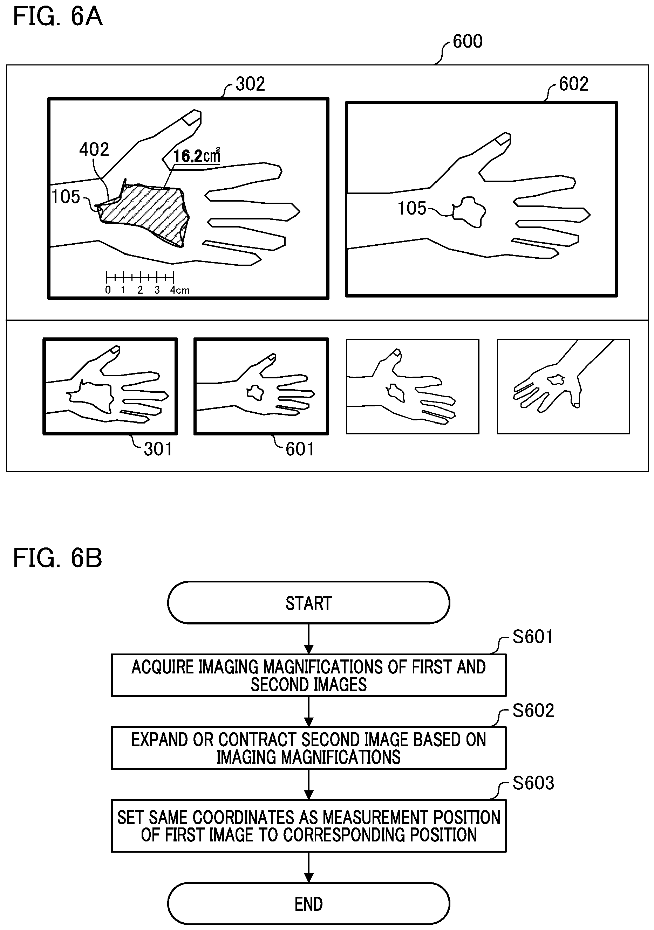

Fig. 6(A) is a diagram illustrating an example of a screen displayed on the output unit 14 after the second image 601 is selected. An expanded image 602 of the second image 601 is displayed in the top right of the screen.

When, in the first image 301 and the second image 601, there is a subject (skin disease part 105) at substantially the same position on the images and only imaging magnifications are different, the corresponding position is calculated through a process illustrated in the flowchart of Fig. 6(B).

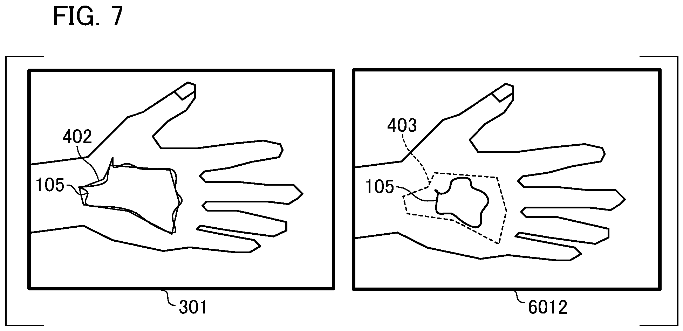

First, in S601 of Fig. 6(B), the control unit 11 acquires the imaging magnifications of the first and second images. Subsequently, in S602, the control unit 11 expands or contracts the second image 601 like an image 6012 based on the imaging magnifications so that the sizes of the subjects of the first image 301 and the second image 601 are substantially the same, as in Fig. 7. The control unit 11 preferably expands or contracts at least one of the first and second images so that sizes of the subjects corresponding in the first and second images are equal. Finally, in S603, the control unit 11 sets the same pixel coordinate position as a measurement position 402 on the first image to a corresponding position 403 of the second image, in the second image 6012 which is expanded or contracted. In this method, the corresponding position can be obtained through a simple process when the imaging magnifications in imaging are different.

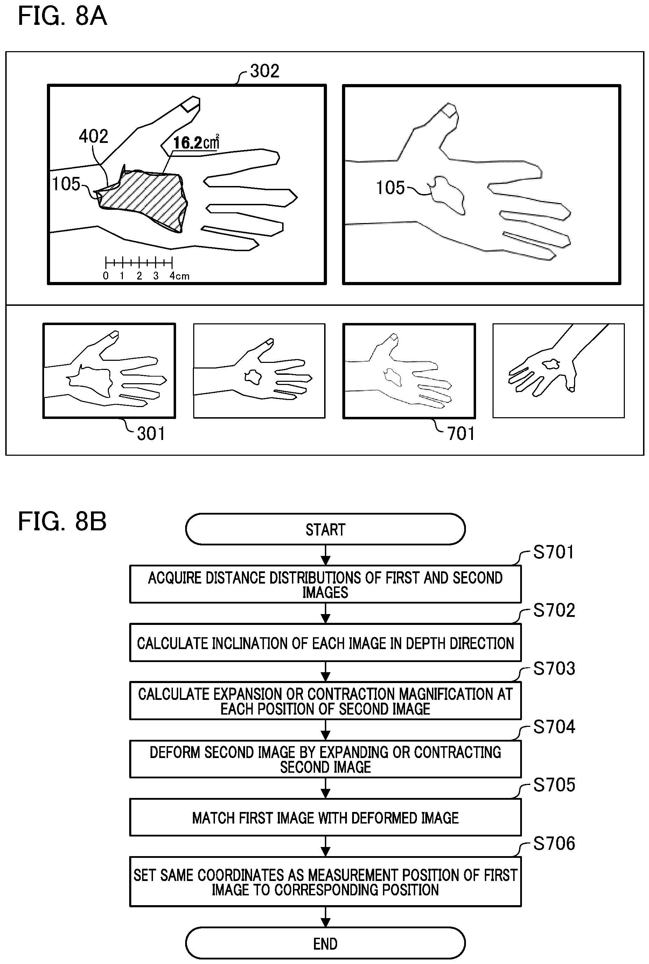

Next, a case in which positions and postures in imaging of the first image 301 and a second image 701 are slightly different as in Fig. 8(A) will be described. In this case, the control unit 11 deforms the second image by expanding or contracting the second image in consideration of a difference between inclinations of the postures based on distance information and calculates the corresponding position on the image, as illustrated in the flowchart of Fig. 8(B). When there is the skin disease part 105 at substantially the same position in the images and the imaging magnifications are different, the corresponding position can be obtained by the above-described method. Therefore, it is assumed that the imaging magnifications are the same.

First, in S701 of Fig. 8(B), the control unit 11 acquires a distance distribution corresponding to each of the first and second images. Subsequently, in S702, the control unit 11 calculates the inclination of the distance distribution of each image in the depth direction. Subsequently, in S703, the control unit 11 calculates an expansion or contraction magnification at each position of the second image using each inclination so that the second image becomes similar to the first image.

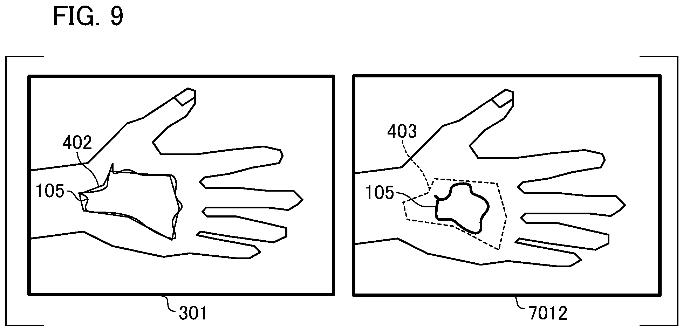

Subsequently, in S704, the control unit 11 deforms the second image 701 like an image 7012 using the magnification calculated in S703 as in Fig. 9. In S705, the control unit 11 compares the second image deformed in S704 with the first image, calculates a position deviation amount between both the images, and corrects the second image. The control unit 11 calculates a position deviation amount, for example, through matching or the like of feature points of the images. Finally, in S603, the control unit 11 sets the same pixel coordinate position as the measurement position 402 on the first image on the second image corrected in S705 to the corresponding position 403 of the second image. In this method, the corresponding position can be obtained through a simple process when the postures in imaging are different.

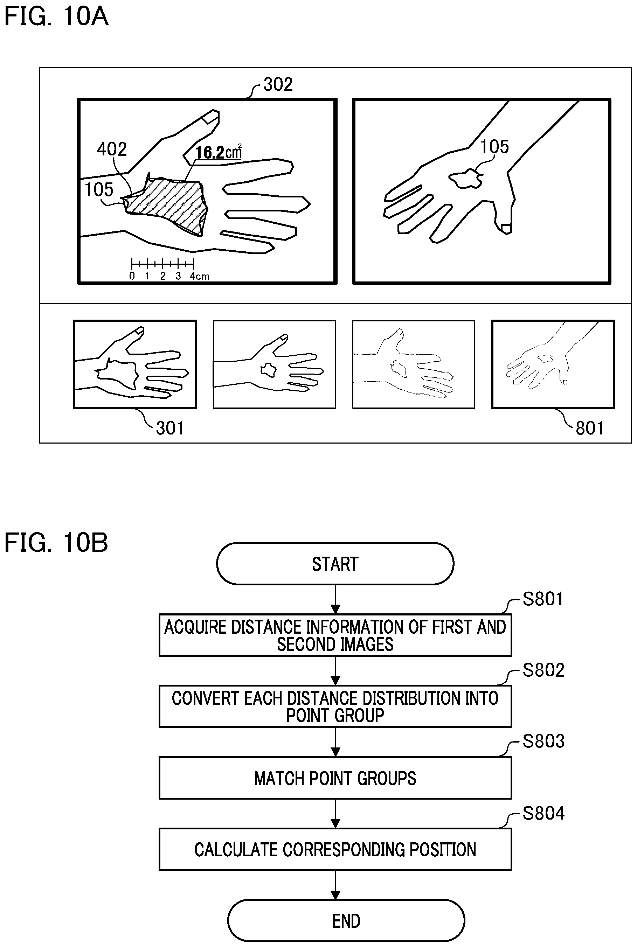

A case in which positions and postures of the first image 301 and the second image 801 in imaging are different as in Fig. 10(A) will be described. In this case, the control unit 11 calculates a corresponding position based on distance information as illustrated in the flowchart of Fig. 10(B).

In S801 of Fig. 10(B), the control unit 11 acquires the distance information corresponding to each of the first and second images. Subsequently, in S802, the control unit 11 converts the distance distributions of the first and second images into point groups based on information regarding a focal distance and a sensor. In step S803, the control unit 11 compares both the point groups, estimates a difference between the postures or positions of the point groups, and obtains a correspondence relation between the point groups. As a scheme of obtaining the correspondence relation between the point groups, a scheme of calculating similarity between the point groups and estimating a difference between the positions or postures, such as an ICP algorithm, can be used.

Finally, in S804, the control unit 11 obtains a point group of the second image corresponding to the measurement point and sets position coordinates on the second image corresponding to the point group to the corresponding position. In this method, the corresponding position can be obtained with high accuracy even when the positions and postures in imaging are significantly different.

A shape or a phase such as a curve state of a joint of an arm or a cure situation of a skin disease part is different at a timing of imaging even in the same subject. When there is a different region obviously between the first and second images, a corresponding position may be obtained excluding distance information or an image corresponding to the region. Calculation accuracy of the corresponding position is improved. A method of detecting the region may be designated by a method similar to the method of detecting the measurement position.

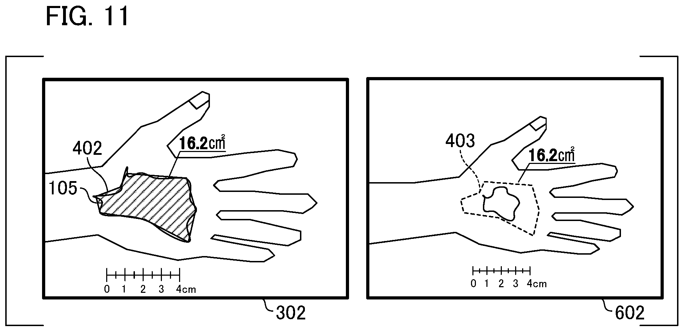

In this case, in S207, the control unit 11 superimposes and displays the information regarding the measurement position of the first image on the corresponding position 403 of the second image as in Fig. 11 on the output unit 14. The control unit 11 preferably adjusts the first measurement information so that lengths or sizes of predetermined subjects in the first and second images are equal and superimposes the first measurement information. As described above, the information regarding the measurement position is information for showing the measurement result 501 such as an area or a length or a measurement portion such as the oblique line region 502 and further a scale or an auxiliary line suggesting the information to the user for easy understanding. The control unit 11 may superimpose and display a memory that can be compared with the first measurement information along with the first measurement information as in Fig. 11. By superimposing and displaying a measurement result of the first image on the same subject of the second image, the user can easily understand a difference between similar subjects captured in two images.

Each image may be deformed and displayed as any image so that the sizes or postures of the subjects of the first and second images become similar or positions of the subjects on the screen become similar. For example, when the sizes or postures of the subjects in the images are different, one or both of the images are deformed and displayed based on the imaging magnifications or the distance distributions as in S602 or S704. When the positions are different, the images or the distance distributions are compared, a position deviation amount between both the images is calculated, and one or both of the images are displaced and displayed. Since phases of the appearances of the first and second images become similar, the user further understands a difference between similar subjects easily.

When a size of a detection position is smaller with respect to the size of a displayed image because of the same reason, an image near the corresponding position of the second image and the detection position of the first image may be expanded and displayed on the output unit 14.

As described above, by superimposing and displaying the information regarding the measurement position of the first image at the corresponding position of the second image, the user can understand a difference in a measurement target between the first and second images more easily.

In the calculation of the corresponding position of the second image, a difference between the sizes, positions, or directions of subjects of both images may be obtained using the distance information of the first and second images. Accordingly, the corresponding position of the second image corresponding to the measurement position of the first image may be calculated.

Accuracy in the calculation of the corresponding position of the second image may be obtained and the obtained accuracy may be superimposed and displayed in the second image.

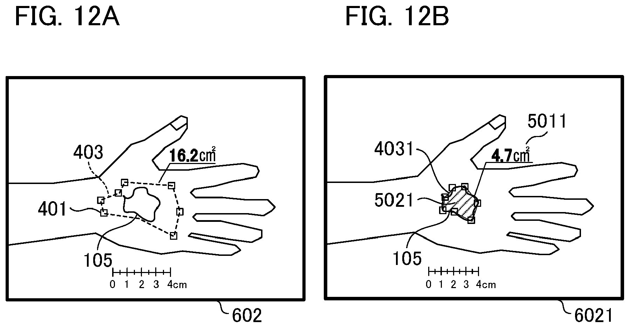

In the embodiment, the example in which the information regarding the measurement position of the first image is superimposed and displayed at the corresponding position of the second image has been described. However, based on the result, the measurement position may be designated on the second image and measurement may be performed. Fig. 12 illustrates the above state.

Fig. 12(A) is a diagram illustrating the expanded second image 602. The node 401 is displayed at the corresponding position 403. The user designates the measurement position of the skin disease part 105 again by operating the node 401.

Fig. 12(B) is a diagram illustrating an image 6021 in which a new measurement position 4031 and information regarding the measurement position 4031 is superimposed. An area 5011 and a measurement region (oblique line portion) 5021 at the measurement position are displayed. The measurement region (oblique line portion) illustrated in Fig. 12(B) is an example of the above-described second measurement information. That is, the control unit 11 preferably further superimposes and displays the second measurement information obtained by measuring a subject in the second image. In this way, the user can designate the measurement position 4031 in the second image more easily by setting the corresponding position 403 obtained in advance as an initial position of a new measurement position.

In the embodiment, the corresponding position of the second image has been calculated after the measurement position of the first image is designated. However, the corresponding positions of the first and second images may be calculated earlier and only a measurable range may be designated as a measurement position. That is, a position at which a subject is measured in the second image may be received within a predetermined range that is based on the first position. Accordingly, it is possible to prevent a detection error of the corresponding position in advance.

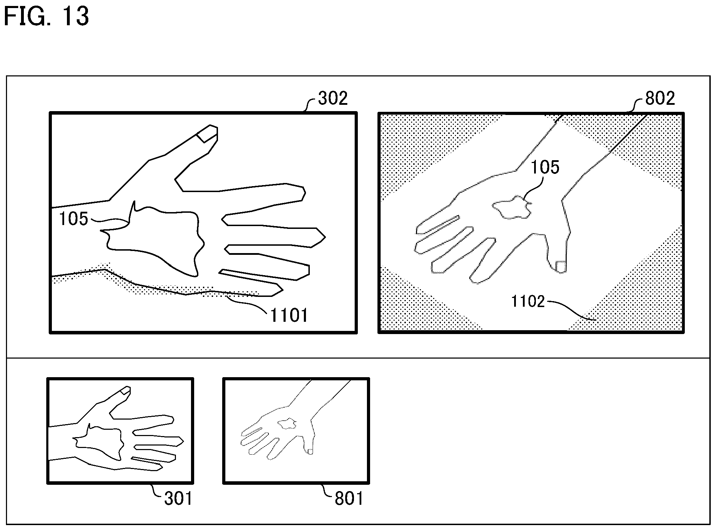

Fig. 13 is a diagram illustrating an example when only a measurable range can be designated as a measurement position. In Fig. 13, the first image 301 and the second image 801 are each selected to be expanded and displayed. In Fig. 13, the first image 301 and the second image 302 are displayed side by side. A region 1101 grayed out in the expanded image 302 of the first image 301 is an unmeasurable region since the region is hidden in the shadow of a hand in the second image 801 and is not captured. Similarly, a region 1102 grayed out in an expanded image 802 of the second image 801 is an unmeasurable region since the region is not captured in the first image 301. Since the user can understand the unmeasurable region at first sight by the grayed-out display, it is possible to prevent a detection error of the corresponding position in advance. In the embodiment, the unmeasurable region is grayed out and displayed, but another method may be used as long as the user can recognize the unmeasurable region.

At this time, after the measurement position of the first image 301 is detected, an image of which a corresponding position is difficult to calculate among a plurality of read images may be grayed out and displayed on the output unit 14. Accordingly, the user can easily understand which image can be compared among the plurality of images.

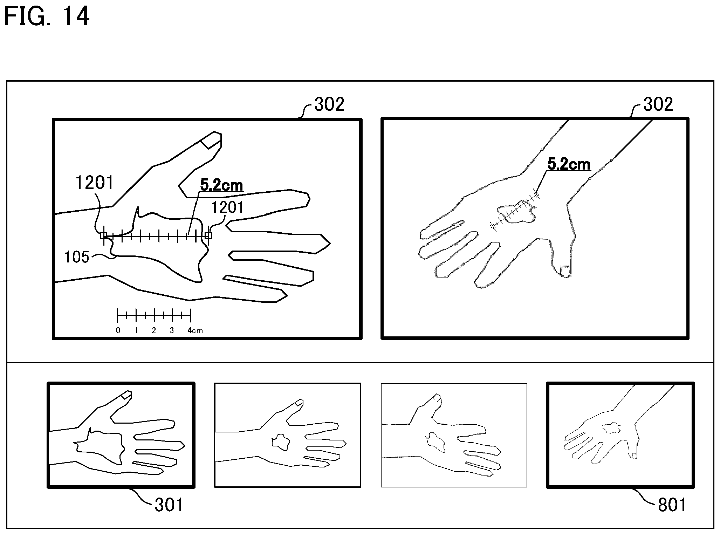

In the embodiment, the area of the skin disease part 105 has been measured. In the present invention, however, any target may be measured as long as the target can be measured based on distance information. For example, the user desires to measure a length of the skin disease part 105 in a direction of the arm, the control unit 11 displays two nodes 1201 indicated by squares in the expanded image 302 of Fig. 14. The user designates the measurement position 402 (a straight line connecting the nodes 1201) by operating the nodes 1201.

Information regarding the measurement position 402 of the first image 301 is superimposed and displayed in the expanded image 302 and the information is further superimposed and displayed at the corresponding position 403 of the expanded image 802 of the second image 801, and thus the user can easily understand a difference between the measurement targets. Examples of the information regarding the measurement position 402 of the first image 301 include the measurement result 501, the scale 503, and the auxiliary line 504 suggested so that the user can easily understand them.

Similarly to the measurement of the area, a new measurement position may be designated using the corresponding position 403 as an initial position in the expanded image 802. The user can designate the measurement position in the second image 801 more easily.

In the embodiment, the number of measurement positions or corresponding positions in one image is only one, but two or more measurement positions may be designated to measure a length, an area, or the like. At the corresponding position captured in the second image 801, information regarding a measurement position is superimposed and displayed. At this time, when the information regarding the measurement position is densely displayed on the output unit 14 and visibility of the subject is inhibited, the user may display only information regarding the measurement position designated through the input unit 10 on the output unit 14.

When a plurality of similar subjects is captured in one image, the user may designate a region of each subject through the input unit 10 and the control unit 11 may consider each of the designated regions as a plurality of images and perform the process according to the present invention.

### (Other Embodiments)

The present invention can also be realized through a process of supplying a program implementing one or more of the functions of the above-described embodiment to a system or a device via a network or a storage medium and causing one or more processors in a computer of the system or the device to read and execute the program. The present invention also be realized by a circuit (for example, an ASIC) implementing one or more functions.

The preferred embodiments of the present invention have been described, but the present invention is not limited to these embodiments and various modifications and changes can be made within the scope of the gist of the present invention.

### (CROSS REFERENCE TO RELATED APPLICATION)

Priority is claimed on Japanese Patent Application No. 2021-205315, filed December 17, 2021, the content of which is incorporated herein by reference.

## Claims

1. An image display device comprising:
acquisition means for acquiring a first image, a second image, and first position information regarding a first position at which a subject in the first image is measured;
display means for displaying the second image on a display device; and
determination means for determining a second position corresponding to the first position in the second image,
wherein the display means displays and superimposes first measurement information obtained by measuring a subject at the first position on the first image at the second position on the second image.

2. The image display device according to claim 1, further comprising designation means for receiving a designation of the first measurement position by a user.

3. The image display device according to claim 1 or 2, wherein the first measurement information is information regarding a length of a line connecting two points including the first position on the first image or information regarding an area of a region including the first position on the first image.

4. The image display device according to any one of claims 1 to 3, wherein the first measurement information is obtained by measuring a length or an area of the subject based on the first image and first distance information in a depth direction corresponding to the first image.

5. The image display device according to claim 3, wherein the first measurement information displayed by the display means includes a frame indicating a region including the first position or a line connecting two points including the first position.

6. The image display device according to any one of claims 1 to 5, wherein the display means adjusts the first measurement information so that lengths or sizes of predetermined subjects in the first and second images are equal, and superimposes the first measurement information on the second image.

7. The image display device according to any one of claims 1 to 6, wherein the display means performs display by expanding or contracting at least one of the first and second images so that sizes of corresponding subjects in the first and second images are equal.

8. The image display device according to any one of claims 1 to 7, wherein the display means further superimposes and displays second measurement information obtained by measuring the subject in the second image.

9. The image display device according to claim 8, wherein the designation means receives a designation of a position at which the subject in the second image is measured within a predetermined range that is based on the first position.

10. The image display device according to any one of claims 1 to 7, wherein the display means superimposes and displays a scale comparable with the first measurement information along with the first measurement information.

11. The image display device according to any one of claims 1 to 8, wherein the first measurement information includes information regarding measurement accuracy when a subject is measured.

12. The image display device according to any one of claims 1 to 9, wherein the display means displays the first and second images side by side.

13. An image display method comprising:
acquiring a first image, a second image, and first position information regarding a first position at which a subject in the first image is measured;
displaying the second image on a display device; and
determining a second position corresponding to the first position in the second image,
wherein display means displays and superimposes first measurement information obtained by measuring a subject at the first position on the first image at the second position on the second image.

14. A program performing:
acquiring a first image, a second image, and first position information regarding a first position at which a subject in the first image is measured;
displaying the second image on a display device; and
determining a second position corresponding to the first position in the second image,
wherein display means displays and superimposes first measurement information obtained by measuring a subject at the first position of the first image at the second position of the second image.

15. A storage medium storing a program performing:
acquiring a first image, a second image, and first position information regarding a first position at which a subject in the first image is measured;
displaying the second image on a display device; and
determining a second position corresponding to the first position in the second image,
wherein display means displays and superimposes first measurement information obtained by measuring a subject at the first position of the first image at the second position of the second image.

## Amended claims

### Amended claims under Art. 19.1 PCT

1. (Amended) An image display device comprising:
acquisition means for acquiring a first image, a second image, and first position information regarding a first position at which a subject in the first image is measured;
display means for displaying the second image on a display device; and
determination means for determining a second position corresponding to the first position in the second image based on distance information corresponding to the first and second images,
wherein the display means displays and superimposes first measurement information obtained by measuring a subject at the first position on the first image at the second position on the second image.

2. The image display device according to claim 1, further comprising designation means for receiving a designation of the first measurement position by a user.

3. The image display device according to claim 1 or 2, wherein the first measurement information is information regarding a length of a line connecting two points including the first position on the first image or information regarding an area of a region including the first position on the first image.

4. The image display device according to any one of claims 1 to 3 wherein the first measurement information is obtained by measuring a length or an area of the subject based on the first image and first distance information in a depth direction corresponding to the first image.

5. The image display device according to claim 3, wherein the first measurement information displayed by the display means includes a frame indicating a region including the first position or a line connecting two points including the first position.

6. The image display device according to any one of claims 1 to 5, wherein the display means adjusts the first measurement information so that lengths or sizes of predetermined subjects in the first and second images are equal, and superimposes the first measurement information on the second image.

7. The image display device according to any one of claims 1 to 6, wherein the display means performs display by expanding or contracting at least one of the first and second images so that sizes of corresponding subjects in the first and second images are equal.

8. The image display device according to any one of claims 1 to 7, wherein the display means further superimposes and displays second measurement information obtained by measuring the subject in the second image.

9. The image display device according to claim 8, wherein the designation means receives a designation of a position at which the subject in the second image is measured within a predetermined range that is based on the first position.

10. The image display device according to any one of claims 1 to 7, wherein the display means superimposes and displays a scale comparable with the first measurement information along with the first measurement information.

11. The image display device according to any one of claims 1 to 8, wherein the first measurement information includes information regarding measurement accuracy when a subject is measured.

12. The image display device according to any one of claims 1 to 9, wherein the display means displays the first and second images side by side.

13. (Amended) An image display method comprising:
acquiring a first image, a second image, and first position information regarding a first position at which a subject in the first image is measured;
displaying the second image on a display device; and
determining a second position corresponding to the first position in the second image based on distance information corresponding to the first and second images,
wherein display means displays and superimposes first measurement information obtained by measuring a subject at the first position on the first image at the second position on the second image.

14. (Amended) A program performing:
acquiring a first image, a second image, and first position information regarding a first position at which a subject in the first image is measured;
displaying the second image on a display device; and
determining a second position corresponding to the first position in the second image based on distance information corresponding to the first and second images,
wherein display means displays and superimposes first measurement information obtained by measuring a subject at the first position on the first image at the second position on the second image.

15. (Amended) A storage medium storing a program performing:
acquiring a first image, a second image, and first position information regarding a first position at which a subject in the first image is measured;
displaying the second image on a display device; and
determining a second position corresponding to the first position in the second image based on distance information corresponding to the first and second images,
wherein display means displays and superimposes first measurement information obtained by measuring a subject at the first position on the first image at the second position on the second image.
